# FASCICULE DE BREVET EUROPEEN

(11) **EP 2 206 532 B1**
(45) Date de publication et mention de la délivrance du brevet: **09.09.2015**
(21) Numéro de dépôt: 09172839.4
(22) Date de dépôt: 13.10.2009
(51) Int. Cl.: A61N 1/37, G01R 29/08, A61N 1/39, G01R 33/28

(54) **Prothèse cardiaque implantable comprenant des moyens de détection de champs magnétiques statiques forts et de mise en sécurité lors d'un examen IRM**
Implantierbarer Herzschrittmacher, der Mittel zum Erkennen von starken statischen Magnetfeldern und zur Sicherung während einer Kernspintomographie umfasst
Implantable medical heart device comprising means for detecting intense static magnetic fields and commuting to safety mode during MRI tests

(30) Priorité: 09.01.2009 FR 0900059
(43) Date de publication de la demande: 14.07.2010
(73) Titulaire: ELA MEDICAL, 92541 Montrouge (FR)
(72) Inventeur: Legay, Thierry, 91640, FONTENAY LES BRIIS (FR)
(74) Mandataire: Dupuis-Latour, Dominique

(56) Documents cités:
- EP-A1- 1 935 450
- WO-A2-01/62142
- US-A1- 2003 144 704
- US-A1- 2003 144 705

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Ces dispositifs comportent un boîtier généralement désigné "générateur", raccordé électriquement et mécaniquement à une ou plusieurs "sondes" munie(s) d'électrodes destinées à venir en contact avec le myocarde sur des sites de recueil des potentiels électriques et/ou d'application des impulsions de stimulation. Ces électrodes peuvent être des électrodes endocavitaires (placées dans une cavité du myocarde en contact avec la paroi de celui-ci), épicardiques (notamment pour définir un potentiel de référence, ou pour appliquer un choc), ou encore intravasculaires (la sonde est par exemple introduite dans le sinus coronaire jusqu'à un emplacement situé face à la paroi du ventricule gauche).

L'invention concerne plus particulièrement les techniques permettant de sécuriser ces dispositifs lorsque le porteur doit être soumis à un examen par imagerie par résonance magnétique (IRM ou MRI).

En effet, un examen IRM est aujourd'hui contre-indiqué aux patients porteurs d'un stimulateur ou défibrillateur cardiaque. Plusieurs types de problèmes en sont la cause :
- un échauffement à proximité des électrodes reliant le générateur au coeur du patient ;
- les forces et couples d'attraction exercés sur le dispositif plongé dans le champ magnétique statique très élevé de l'appareil IRM ;
- un comportement imprédictible du dispositif lui-même, du fait de l'exposition à ces champs magnétiques extrêmes.

La présente invention a pour objet d'apporter une solution au troisième type de problème.

Il est en effet souhaitable que lors de l'exposition au champ électromagnétique (statique et alternatif) généré par l'imageur IRM, le dispositif ait un comportement documenté et connu au préalable.

Les principaux problèmes susceptibles d'affecter le dispositif dans une telle situation sont :
- une détection erratique du champ statique fort généré par l'imageur IRM, notamment pour les dispositifs pourvus d'un détecteur de champ magnétique de type interrupteur à lame souple (ILS). Un tel interrupteur est utilisé pour détecter la présence à proximité de l'implant d'un aimant permanent, habituellement utilisé par un praticien pour mettre le dispositif dans un mode de fonctionnement sécurisé spécifique, par exemple en cas d'utilisation d'un bistouri électrique, ou encore pour évaluer l'usure de la pile du dispositif (en mode à l'aimant, la fréquence de stimulation est fixe et reflète le niveau de charge de la pile). Un ILS, conçu pour détecter des champs magnétiques statiques relativement faibles, présente en revanche un comportement totalement imprévisible dans un environnement d'imagerie IRM, où les champs magnétiques sont souvent plus de mille fois supérieurs à ceux d'un aimant permanent ;
- la dégradation de ses performances intrinsèques ;
- le fait que les signaux dynamiques émis par l'imageur IRM peuvent être détectés par le dispositif et interprétés à tort comme des signaux cardiaques.

À ce dernier égard, il y a lieu de prendre en compte le fait que pendant toute la durée de l'examen - qui peut se prolonger plusieurs minutes -, le dispositif doit néanmoins rester fonctionnel et assurer sans discontinuité si nécessaire la stimulation du myocarde.

Il est donc nécessaire de disposer de moyens de détection et de gestion d'une telle situation, assurant les fonctions suivantes :
- indication au dispositif que le patient va être soumis à un examen IRM ;
- inhibition des circuits du dispositif susceptibles d'être perturbés par les champs électromagnétiques émis par l'imageur IRM ;
- fonctionnement dans un mode de stimulation dédié, adapté au patient et compatible avec les champs électromagnétiques produits par l'imageur.

L'invention concerne plus particulièrement la première fonction, qui est une étape préalable à la mise en oeuvre des deux autres.

Une première approche consiste, en utilisant une commande spéciale envoyée depuis un programmateur externe, à signifier au dispositif qu'il va être exposé à des champs de type IRM et qu'il doit en conséquence adopter une configuration particulière, tant pour son fonctionnement propre que pour la stimulation délivrée.

La difficulté de cette approche tient au fait qu'elle nécessite de disposer d'un programmateur avant l'examen, qu'elle requiert donc la présence d'un praticien qualifié, et surtout qu'elle impose après l'examen une reprogrammation du dispositif pour le replacer dans son mode de fonctionnement initial, sans quoi le patient pourrait quitter le centre d'IRM avec un dispositif opérant dans un mode de fonctionnement non optimal pour la vie courante. Il existe donc toujours un risque que le centre pratiquant l'IRM oublie de reprogrammer la prothèse avant le départ du patient.

L'autre approche consiste à équiper le dispositif de moyens de détection automatique de champ magnétique.

On a indiqué plus haut que les prothèses cardiaques sont généralement équipées d'un détecteur de champ faible, sensible à la présence d'un aimant disposé à proximité du dispositif pour placer celui-ci dans un "mode à l'aimant" particulier. Les détecteurs de champ faible les plus courants utilisent un ILS (qui présente l'inconvénient d'une taille excessive, et d'un comportement imprédictible face à des champs statiques forts de type IRM), ou encore des capteurs intégrés tels que des composants de type MAGFET comme cela est par exemple décrit dans le WO 94/12238 A1 (qui présentent l'inconvénient de prélever de l'énergie pour fonctionner), ou encore des capteurs de type microsystème électromécanique MEMS, comme cela est par exemple décrit dans le FR 2 805 999 A1.

Ces divers capteurs, utilisés pour détecter un champ faible (typiquement de l'ordre de 1,5 mT) sont inadaptés à la détection des champs critiques forts tels que ceux produits par les imageurs IRM (typiquement compris entre 0,5 T et 3 T, voire plus) qui sont donc près de mille fois plus élevés, et se situent dans des zones de réponse non linéaires des capteurs conventionnels. Un capteur de champ faible risque donc être "sourd" à la présence d'un champs fort.

Des techniques particulières ont été proposées pour détecter des champs magnétiques statiques forts de type IRM (ordre de grandeur du tesla).

Le US 2007/191914 A1 décrit ainsi un dispositif dans lequel la présence d'un champ magnétique statique fort est détectée par analyse de l'impédance d'un composant inductif, par exemple de l'une des bobines d'un régulateur inductif à découpage : la présence d'un champ magnétique fort a pour effet de saturer le noyau de ce composant, provoquant un changement d'impédance qui peut être signalé au dispositif.

Le WO 2006/124481 A2 décrit une autre technique, consistant à détecter la présence d'un champ IRM par mesure de la tension recueillie aux bornes d'une antenne de télémétrie ainsi que sur la sonde.

Ces divers dispositifs ne permettent pas de pallier toutes les difficultés exposées plus haut, encore moins avec le très haut degré de fiabilité requis pour un dispositif implanté destiné à fonctionner de façon entièrement automatique.

Le EP 1 935 450 A1 décrit une autre technique encore, consistant à utiliser des capteurs de type résistance magnétique géante (RMG ou GMR) associés en un pont de Wheatstone. Ce pont va jouer un rôle de capteur unique mixte champ fort/champ faible : en effet, l'équilibre du pont se trouve plus ou moins altéré selon l'amplitude du champ, et les modifications résultantes de la tension différentielle peuvent être analysées par un convertisseur placé en sortie du pont pour donner une estimation globale du niveau de champ.

Le but de l'invention est de proposer un dispositif pourvu d'un mode automatique de détection de l'exposition à des champs magnétiques de type IRM, permettant au dispositif d'entrer dans un mode de fonctionnement sécurisé s'il détecte la présence d'un tel champ, et surtout qui lui permette aussi de quitter de façon automatique ce mode de fonctionnement sécurisé dès qu'il détecte la disparition du champ électromagnétique.

L'invention a également pour but de proposer un tel dispositif qui puisse assurer une distinction entre champ faible (de type aimant permanent) et champ fort (de type IRM) et prendre les actions appropriées dans l'un ou l'autre cas, en plaçant le dispositif dans des modes de fonctionnement sécurisés respectifs distincts, adaptés à la situation correspondante.

Essentiellement, l'invention propose de mettre en oeuvre concurremment deux capteurs de sensibilité différente, le capteur le moins sensible (pour la détection des champs forts) n'étant activé que de façon conditionnelle, sur détection d'un champ par le capteur le plus sensible (pour la détection des champs faibles).

Après détection d'un champ fort, le dispositif se placera dans un mode de fonctionnement spécifique qui le rend insensible au champ produit par l'imageur IRM, ce mode de fonctionnement spécifique étant distinct du "mode à l'aimant" conventionnel, de manière à mieux adapter le fonctionnement à la situation particulière du patient dont on sait qu'il est en train de subir un examen IRM.

L'invention propose à cet effet un dispositif médical actif implantable d'un type connu d'après le EP 1 935 450 A1 précité, comprenant les éléments énoncés dans le préambule de la revendication 1. La partie caractérisante expose les éléments propres à la présente invention. Les sous-revendications visent des formes de réalisation particulières, avantageuses.

On va maintenant décrire un exemple de mise en oeuvre de l'invention, en référence aux dessins annexés où les mêmes références numériques désignent d'une figure à l'autre des éléments identiques ou fonctionnellement semblables.

La Figure 1 est une vue schématique d'un générateur de dispositif implantable, montrant les éléments essentiels de ce dispositif.

La Figure 2 est un synoptique du fonctionnement du dispositif selon l'invention.

En ce qui concerne ses aspects logiciels, l'invention peut être mise en oeuvre par une modification matérielle simple et une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'adaptation de ces appareils à la mise en oeuvre des fonctions de l'invention est à la portée de l'homme du métier, et elle ne sera pas décrite en détail.

L'invention peut notamment être appliquée aux dispositifs implantables tels que ceux des familles *Reply et Paradym* produits et commercialisés par ELA Médical, Montrouge, France. Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous.

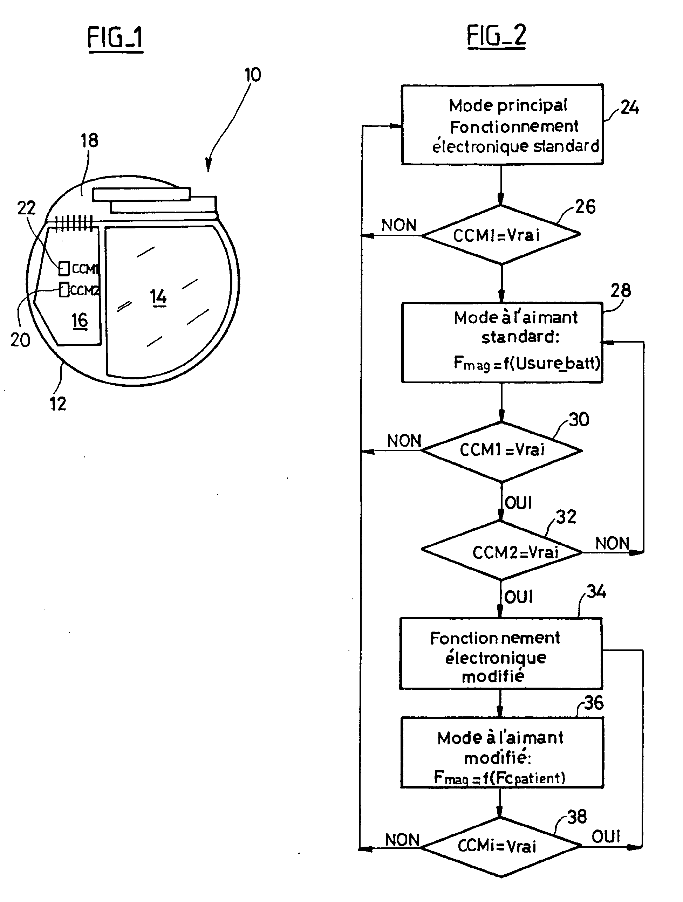

Sur la Figure 1, la référence 10 désigne de façon générale le générateur d'un dispositif implantable de type stimulateur cardiaque.

Cet implant comprend un boîtier 12 logeant une pile d'alimentation 14 et un circuit électronique 16 relié à un connecteur 18 sur lequel pourront être branchées une ou plusieurs sondes pourvues, à leur autre extrémité, d'électrodes de détection/stimulation.

De façon caractéristique de l'invention, le dispositif est pourvu de deux capteurs de champ magnétique 20, 22 mettant en oeuvre des technologies différentes et destinés à détecter des champs statiques d'intensités très différentes.

L'un des capteurs, ci-après désigné CCM1, est un capteur de champ magnétique faible (typiquement un champ de l'ordre du millitesla). Sa technologie peut être l'une de celles couramment utilisées pour la détection d'un aimant permanent approché de l'implant en vue de placer celui-ci dans un "mode à l'aimant" spécifique. Pour ce choix technologique, on évitera toutefois les capteurs de type ILS comprenant des éléments métalliques, dont le comportement est imprédictible en présence de champs forts. Le capteur pourra être choisi parmi les composants de type : bobine dont le noyau sature en présence d'un champ magnétique faible, résistance magnétique géante (RMG), capteur à effet Hall, etc. Le capteur CCM1 est un capteur qui, normalement, est déjà présent dans le dispositif et peut être utilisé sans modification matérielle.

Le capteur de champ fort CCM2, quant à lui, est un capteur additionnel. On choisira de préférence un capteur sur silicium de type MAGFET, qui peut être directement intégré à la puce électronique du circuit du dispositif, donc sans perte significative de surface disponible sur le circuit 16.

Le MAGFET est un transistor MOS à effet de champ comportant plusieurs drains, la zone d'inversion dans le canal du transistor créée par la polarisation étant utilisée comme zone de déflection des porteurs lors de l'application d'un champ magnétique perpendiculaire au plan du canal. De préférence, le MAGFET est réalisé à partir de MOS de type N, car leur meilleure mobilité améliore la sensibilité. La sensibilité est également fonction de divers paramètres géométriques tels que la distance entre les deux drains, la longueur et la largeur du canal, et dépend également des conditions de polarisation électrique.

Le capteur pourra comprendre un ou plusieurs de ces MAGFETs, associés à un système de détection de courant permettant de convertir le champ magnétique en un signal ou grandeur électrique telle que courant, tension ou fréquence.

Un tel capteur de type MAGFET présente la particularité de consommer de l'énergie pour fonctionner, et d'autre part sa sensibilité est relativement faible (variations de l'ordre de quelques pourcents par tesla), ce qui le rend particulièrement bien adapté à la détection de champs magnétiques élevés, typiquement des champs supérieurs à 250 mT, de l'ordre de 0,5 à 3 T ou plus.

Le dispositif 10 dispose ainsi de deux capteurs de champ :
- un capteur CCM1 de champ magnétique statique faible, typiquement supérieur ou égal à 1 mT, et
- un capteur CCM2 ne réagissant qu'aux champs statiques forts, typiquement supérieurs à 0,25 T.

La Figure 2 illustre la manière dont ces deux capteurs sont utilisés en combinaison.

Au départ, le dispositif est en mode de fonctionnement standard, non modifié (bloc 24).

Si le capteur de champ faible CCM1 détecte un signal (test 26), alors le dispositif passe en "mode à l'aimant" classique (bloc 28). Ce mode en lui-même bien connu, ci-après dénommé "mode à l'aimant standard" consiste essentiellement à stimuler le patient en mode DOO avec une fréquence de stimulation dépendant du niveau d'usure de la pile (ce qui permet de tester ce niveau).

Si, en outre, le capteur de champ fort CCM2 détecte un signal (test 32), alors le fonctionnement du dispositif est modifié de la manière que l'on va expliquer ci-après (blocs 34, 36).

Si seul CCM2 détecte un signal, on considère qu'il s'agit d'un artefact dû à l'implémentation de ce capteur, et aucun changement n'est opéré dans le fonctionnement de la prothèse.

Le capteur CCM2 n'est donc scruté que si le capteur CCM1 a déjà détecté un champ : ce principe permet, de façon avantageuse, de limiter la consommation de courant en n'alimentant l'électronique d'exploitation du capteur de champ fort CCM2 que si le capteur de champ faible CCM1 a déjà donné un résultat positif.

Si les deux capteurs CCM1 et CCM2 détectent simultanément un champ magnétique, on considère alors que l'on est en présence d'un champ propre à un appareil IRM et l'on place le dispositif dans un "mode à l'aimant modifié" (bloc 36) adapté à cette situation.

Avantageusement, dans ce "mode à l'aimant modifié" la fréquence de stimulation n'est plus indexée sur l'usure de la pile (comme dans le "mode à l'aimant standard"), mais sur la moyenne du rythme cardiaque du patient, légèrement augmentée (d'une valeur fixe, par exemple de 10 bpm, ou d'un pourcentage, par exemple de 10 %) : cette légère augmentation de la fréquence cardiaque de stimulation permet d'assurer une non-compétition du rythme stimulé (permanent) avec le rythme spontané du patient. On peut ainsi obtenir un fonctionnement sûr, avec inhibition de la fonction de détection pour éviter de détecter les champs électromagnétiques dynamiques émis par l'imageur IRM, mais en gardant une marge de sécurité permettant une stimulation du patient en toute sécurité (à la différence d'un mode à l'aimant standard).

D'autres modifications peuvent être opérées dans le fonctionnement du stimulateur lorsque celui-ci est placé en "mode à l'aimant modifié" (bloc 34) :
- dans le cas d'un défibrillateur implantable, il est souhaitable d'interdire la délivrance de chocs pendant toute la durée de l'examen ;
- il est par ailleurs souhaitable de désactiver un certain nombre de circuits électroniques du dispositif utilisant directement ou indirectement des propriétés magnétiques, en particulier les circuits de télémétrie RF et les alimentations à découpage (les systèmes d'alimentation seront alors basés sur des régulateurs linéaires de tension ou des convertisseurs capacitifs, consommant certes plus d'énergie, mais insensibles aux effets des champs magnétiques).

Le "mode à l'aimant modifié" que l'on vient de décrire est maintenu tant que dure l'examen IRM.

La fin de cet examen peut être détectée (test 38) par le changement d'état de l'un ou l'autre des capteurs CCM1 ou CCM2 :
- si c'est le capteur de champ faible CCM1 qui est utilisé pour ce test, le retour au mode de fonctionnement normal interviendra lorsque plus aucun champ magnétique statique, même faible, ne sera détecté par le dispositif, c'est-à-dire, concrètement, quand le patient sortira de la salle d'examen où se trouve l'imageur ;
- si c'est le capteur de champ fort CCM2 qui est utilisé pour ce test, le retour au mode de fonctionnement normal interviendra plus tôt, dès que le patient sortira du tube de l'imageur IRM. En revanche, dans la mesure où ce capteur sera utilisé pendant toute la durée de l'examen, la consommation énergétique du dispositif sera accrue (consommation du MAGFET qui doit être alimenté pour fonctionner).

On notera que la configuration sécurisée de "mode à l'aimant modifié" que l'on vient de décrire peut être appliquée non seulement aux examens IRM, mais également utilisée comme protection dans divers autres environnements électromagnétiques créés par des appareils médicaux tels que bistouri électrique, dispositifs de stimulation électrique transcutanée des nerfs (TENS), etc. ou encore des équipements de la vie courante tels que portique antivol, dispositifs de surveillance électrique des articles (EAS), etc.

## Revendications

1. Un dispositif médical actif implantable (10) du type prothèse cardiaque de stimulation, resynchronisation et/ou défibrillation, comprenant :
- un circuit électronique de détection/stimulation (16) ;
- un capteur de champ faible (22), apte à détecter la présence d'un champ magnétique statique d'un niveau correspondant à celui susceptible d'être émis par un aimant permanent approché au voisinage du dispositif ;
- des moyens de détection de champ IRM, comprenant un capteur de champ fort (20) apte à détecter la présence d'un champ magnétique statique d'un niveau correspondant à celui susceptible d'être émis par un imageur IRM ; et
- des moyens de mise en sécurité, aptes à placer le circuit électronique dans un mode IRM protégé spécifique,
**caractérisé en ce que** :
- le capteur de champ fort est un capteur différent du capteur de champ faible, et est un capteur activable de façon conditionnelle,
- le dispositif comprend en outre des moyens d'activation sélective du capteur de champ fort (20) opérant en réponse au capteur de champ faible, aptes à n'activer le capteur de champ fort que sur détection d'un champ magnétique par le capteur de champ faible (22), et
- les moyens de mise en sécurité IRM sont aptes à placer le circuit électronique dans ledit mode IRM protégé spécifique seulement lorsque l'un et l'autre des capteurs de champ fort et faible (20, 22) détectent la présence d'un champ magnétique.

2. Le dispositif de la revendication 1, dans lequel le capteur de champ fort (20) est un capteur apte à détecter la présence d'un champ magnétique statique d'un niveau d'au moins 0,25 T.

3. Le dispositif de la revendication 1, dans lequel le capteur de champ faible (22) est un capteur apte à détecter la présence d'un champ magnétique statique d'un niveau d'au moins 1 mT.

4. Le dispositif de la revendication 1, dans lequel le capteur de champ fort (20) est un capteur alimenté, qui n'est alimenté que sur détection d'un champ magnétique par le capteur de champ faible.

5. Le dispositif de la revendication 1, dans lequel le capteur de champ faible (22) est un capteur choisi dans le groupe formé par : bobine dont le noyau tend à saturer en présence d'un champ magnétique faible, résistance magnétique géante RMG, capteur à effet Hall.

6. Le dispositif de la revendication 1, dans lequel le capteur de champ fort (20) est un capteur intégré de type MAGFET.

7. Le dispositif de la revendication 1, dans lequel les moyens de mise en sécurité sont des moyens aptes à :
- sur détection d'un champ magnétique par le capteur de champ faible :
• mettre le circuit dans un mode de fonctionnement protégé standard de type mode à l'aimant sans détection,
• et activer le capteur de champ fort ; et
- sur détection d'un champ magnétique également par le capteur de champ fort :
• inhiber les organes du dispositif sensibles aux effets délétères d'une exposition aux champs magnétiques statique et radiofréquence émis par l'imageur IRM,
• et modifier au moins un paramètre dudit mode à l'aimant.

8. Le dispositif de la revendication 7, dans lequel :
- le mode à l'aimant standard est un mode dans lequel le circuit électronique de détection/stimulation est apte à produire des impulsions de stimulation à une première fréquence prédéterminée, fonction du niveau de charge de la pile d'alimentation du dispositif ; et
- le mode à l'aimant modifié est un mode dans lequel le circuit électronique de détection/stimulation est apte à produire des impulsions de stimulation à une seconde fréquence prédéterminée, indépendante du niveau de charge de la pile d'alimentation du dispositif.

9. Le dispositif de la revendication 8, dans lequel ladite seconde fréquence prédéterminée est une fréquence fonction du rythme cardiaque moyen antérieur avant détection de la présence d'un champ magnétique par l'un et l'autre des capteurs de champ fort et faible.

10. Le dispositif de la revendication 9, dans lequel ladite seconde fréquence prédéterminée est une fréquence majorée par rapport audit rythme cardiaque moyen antérieur.

## Patentansprüche

1. Aktive implantierbare medizinische Vorrichtung (10) vom Typ Herzschrittmacher zur Stimulation, Resynchronisation und/oder Defibrillation, umfassend:
- eine elektronische Schaltung zur Erkennung/Stimulation (16);
- einen Schwachfeldsensor (22), der geeignet ist, die Gegenwart eines statischen Magnetfelds mit einem Betrag zu erfassen, der dem entspricht, der von einem in die Nähe der Vorrichtung gebrachten Permanentmagneten emittiert werden kann;
- Mittel zum Erfassen des MRT-Felds, umfassend einen Starkfeldsensor (20), der geeignet ist, die Gegenwart eines statischen Magnetfelds mit einem Betrag zu erfassen, der dem entspricht, der von einem MRT-Bildgeber emittiert werden kann; und
- Mittel zur Sicherung, die geeignet sind, die elektronische Schaltung in einem spezifischen, geschützten MRT-Modus anzuordnen,
**dadurch gekennzeichnet, dass**:
- der Starkfeldsensor ein Sensor ist, der sich vom Schwachfeldsensor unterscheidet und ein Sensor ist, der bedingt aktivierbar ist,
- die Vorrichtung ferner Mittel zur selektiven Aktivierung des Starkfeldsensors (20) umfasst, die in Abhängigkeit vom Schwachfeldsensor arbeiten, die geeignet sind, den Starkfeldsensor erst nach dem Erkennen eines Magnetfelds durch den Schwachfeldsensor (22) zu aktivieren, und
- die Mittel zur MRT-Sicherung geeignet sind, die elektronische Schaltung erst in dem spezifischen geschützten MRT-Modus anzuordnen, wenn sowohl Starkfeld- als auch Schwachfeldsensor (20, 22) die Gegenwart eines Magnetfelds erkennen.

2. Vorrichtung nach Anspruch 1, wobei der Starkfeldsensor (20) ein Sensor ist, der die Gegenwart eines statischen Magnetfeldes mit einem Betrag von wenigstens 0,25 T erkennen kann.

3. Vorrichtung nach Anspruch 1, wobei der Schwachfeldsensor (22) ein Sensor ist, der die Gegenwart eines statischen Magnetfeldes mit einem Betrag von wenigstens 1 mT erkennen kann.

4. Vorrichtung nach Anspruch 1, wobei der Starkfeldsensor (20) ein versorgter Sensor ist, der erst bei Erkennen eines Magnetfelds durch den Schwachfeldsensor versorgt wird.

5. Vorrichtung nach Anspruch 1, wobei der Schwachfeldsensor (22) ein Sensor ist, der aus der Gruppe gewählt ist, die gebildet ist aus: Spule, deren Kern in Gegenwart eines schwachen Magnetfeldes zur Sättigung neigt, Riesenmagnetowiderstand (GMR), Hall-Effekt-Sensor.

6. Vorrichtung nach Anspruch 1, wobei der Starkfeldsensor (20) ein integrierter MAGFET-Sensor ist.

7. Vorrichtung nach Anspruch 1, wobei die Mittel zur Sicherung Mittel sind, die geeignet sind:
- bei Erkennen eines Magnetfelds durch den Schwachfeldsensor:
• die Schaltung in einen geschützten Standardbetriebsmodus vom Typ Magnetmodus ohne Erkennung zu versetzen;
• und den Starkfeldsensor zu aktivieren; und
- bei Erkennen eines Magnetfeld ebenfalls durch den Starkfeldsensor:
• die Organe der Vorrichtung zu sperren, die für schädliche Wirkungen der Exposition an statische und hochfrequente Magnetfelder, die von dem MRT-Bildgeber emittiert werden, anfällig sind,
• und mindestens einen Parameter des Magnetmodus zu modifizieren.

8. Vorrichtung nach Anspruch 7, wobei:
- der Standard-Magnetmodus ein Modus ist, in dem die elektronische Schaltung zur Erkennung/Stimulation geeignet ist, Stimulationsimpulse mit einer ersten vorgegebenen Frequenz zu erzeugen, die vom Ladungsniveau der Versorgungsbatterie der Vorrichtung abhängig ist; und
- der modifizierte Magnetmodus ein Modus ist, in dem die elektronische Schaltung zur Erkennung/Stimulation geeignet ist, Stimulationsimpulse mit einer zweiten vorgegebenen Frequenz zu erzeugen, die vom Ladungsniveau der Versorgungsbatterie der Vorrichtung unabhängig ist.

9. Vorrichtung nach Anspruch 8, wobei die zweite vorgegebene Frequenz eine Frequenz ist, die vom vorherigen mittleren Herzrhythmus vor dem Erkennen der Gegenwart eines Magnetfelds sowohl durch den Starkfeld- als auch den Schwachfeldssensor abhängig ist.

10. Vorrichtung nach Anspruch 9, wobei die zweite vorgegebene Frequenz eine Frequenz ist, die in Bezug auf den vorherigen mittleren Herzrhythmus erhöht ist.

## Claims

1. An active implantable medical device (10) such as a cardiac prosthesis for stimulation, resynchronization and/or defibrillation, comprising:
- a detection/stimulation electronic circuit (16);
- a low-field sensor (22), adapted to detect the presence of a static magnetic field of a level corresponding to that liable to be emitted by a permanent magnet brought in the vicinity of the device;
- MRI field-detection means, comprising a high-field sensor (20) adapted to detect the presence of a static magnetic field of a level corresponding to that liable to be emitted by a MRI imaging system; and
- safety means, adapted to place the electronic circuit in an specific protected MRI mode,
**characterized in that**:
- the high-field sensor is a sensor different from the low-field sensor, and is a conditionally-activatable sensor,
- the device further comprises means for the selective activation of the high-field sensor (20) operating in response to the low-field sensor, adapted to activate the high-field sensor only upon detection of a magnetic field by the low-field sensor (22), and
- the MIR safety means are adapted to place the electronic circuit in said specific protected MRI mode only when both high- and low-field sensors (20, 22) detect the presence of a magnetic field.

2. The device of claim 1, wherein the high-field sensor (20) is a sensor adapted to detect the presence of a static magnetic field of a level of at least 0.25T.

3. The device of claim 1, wherein the low-field sensor (22) is a sensor adapted to detect the presence of a static magnetic field of a level of at least 1 mT.

4. The device of claim 1, wherein the high-field sensor (20) is a power-supplied sensor, which is power supplied only upon detection of a magnetic field by the low-field sensor.

5. The device of claim 1, wherein the low-field sensor (22) is a sensor chosen from the group formed by: coil whose core tends to be saturated in the presence of a low magnetic field, giant magnetoresistance (GMR), Hall-effect sensor.

6. The device of claim 1, wherein the high-field sensor (20) is an integrated sensor of the MAGFET type.

7. The device of claim 1, wherein the safety means are means adapted to:
- upon detection of a magnetic field by the low-field sensor:
. place the circuit in a standard protected operation mode of the magnet mode type with no detection,
. and activate the high-field sensor; and
- upon detection of a magnetic field also by the high-field sensor :
. inhibit the elements of the device sensitive to the deleterious effects of an exposition to the static and radiofrequency magnetic fields emitted by the MRI imaging system,
. and modify at least one parameter of said magnet mode.

8. The device of claim 7, wherein:
- the standard magnet mode is a mode in which the detection/stimulation electronic circuit is able to produce stimulation pulses at a first predetermined frequency, function of the charge level of the supply battery of the device; and
- the modified magnet mode is a mode in which the detection/stimulation electronic circuit is able to produce stimulation pulses at a second predetermined frequency, independent of the charge level of the supply battery of the device.

9. The device of claim 8, wherein said second predetermined frequency is a frequency function of the previous average heart rhythm before detection of the presence of a magnetic field by both low- and high-field sensors.

10. The device of claim 9, wherein said second predetermined frequency is an increased frequency with respect to said previous average heart rhythm.
